# EUROPEAN PATENT SPECIFICATION

(11) **EP 3 965 654 B1**
(45) Date of publication and mention of the grant of the patent: **17.07.2024**
(21) Application number: 20718335.1
(22) Date of filing: 17.04.2020
(51) Int. Cl.: A61B 5/103, A61B 5/00

(54) **SENSOR ELEMENT, SENSOR ARRANGEMENT, SENSOR SYSTEM AND METHOD FOR DETECTING A FORCE BETWEEN A FOOT AND A SUPPORTING SURFACE**
SENSORELEMENT, SENSORANORDNUNG, SENSORSYSTEM UND VERFAHREN ZUM ERFASSEN EINER KRAFT ZWISCHEN EINEM FUSS UND EINER STÜTZFLÄCHE
ÉLÉMENT DE CAPTEUR, AGENCEMENT DE CAPTEUR, SYSTÈME DE CAPTEUR ET PROCÉDÉ POUR DÉTECTER UNE FORCE ENTRE UN PIED ET UNE SURFACE DE SUPPORT

(30) Priority: 06.05.2019 EP 19172791
(43) Date of publication of application: 16.03.2022
(73) Proprietor: MCG Motion Capture GmbH, 14471 Potsdam (DE)
(72) Inventor: ERTELT, Thomas, 12557 Berlin (DE); KWIATEK, Andre, 12159 Berlin (DE); VÖLCKERS, Oliver, 14055 Berlin (DE)
(74) Representative: Maiwald GmbH
(86) International application number: PCT/EP2020/060875
(87) International publication number: WO 2020/224937

(56) References cited:
- US-A1- 2013 213 145
- US-A1- 2014 144 251
- US-A1- 2016 287 089

## Description

### FIELD OF THE DISCLOSURE

The present disclosure relates to the field of gait analysis, and in particular to a sensor element, a sensor arrangement, a sensor system and a method for detecting a force between a foot and a supporting surface.

### BACKGROUND OF THE DISCLOSURE

Gait analysis may, for example, be used as an instrument of clinical diagnostics. It has been found that musculoskeletal, connective tissue and neurological diseases account for approximately 41% of all approved rehabilitation services. Arthritic diseases and damages as well as so-called "Running Related Injuries" (RRI) are of outstanding importance under the aspect of gait-associated disorders due to their frequency of occurrence. At the same time, however, chronic diseases such as neuropathic/ischemic pathogenesis and chronic unspecific back pain are becoming increasingly important. What all phenomena have in common is that they are related to gait technique. While a direct connection can be proven in some cases, there seems to be at least an indirect connection with gait in numerous diseases and injuries that are still unexplained today. Here it is often not clear whether the observed connections are causal or compensatory. In order to achieve sustainable improvements in medical care, these questions must be clarified on a large scale and by means of longitudinal examinations, which cannot yet be carried out to the appropriate quality and extent due to the current lack of technical possibilities.

The gait analysis thus represents an instrument of clinical diagnostics, which is currently carried out primarily via complex and cost-intensive laboratory systems. At the same time, the course of many injuries and diseases is completely unclear due to the available laboratory systems.

A known technology for mobile gait analysis systems outside the laboratory is an FSR (Force Sensing Resistor) based system. Basically, so-called FSR sensors consist of two plastic/polymer membranes which may be separated from each other by a thin gap of non-conductive materials, an air gap etc., depending on their constructive design. One of the membranes usually contains two sets of electrodes, the other is printed with a semi-conductive ink. The composition of the three components enables the resistance of the components to change predictably as far as possible by applying a defined force to the sensor surface. Conductive and non-conductive materials are usually applied to the carrier film by screen printing. Here, a semiconducting ink is used, which may essentially consists of a carbon black or carbon-graphite mixture. In addition, substrates may be added for insulation, for reasons of adhesion with other materials and to increase flexibility. The effect of a force on the surface of the detection film causes mechanical deformation of the ink as a result of compression. The conductive particles of the matrix then touch the electrodes, altering the resistance of the sensor film. The higher the applied force, the more contacts or resistance bridges can be closed. The result is a resistance ratio inversely proportional to the force and represents a non-linear signal characteristics.

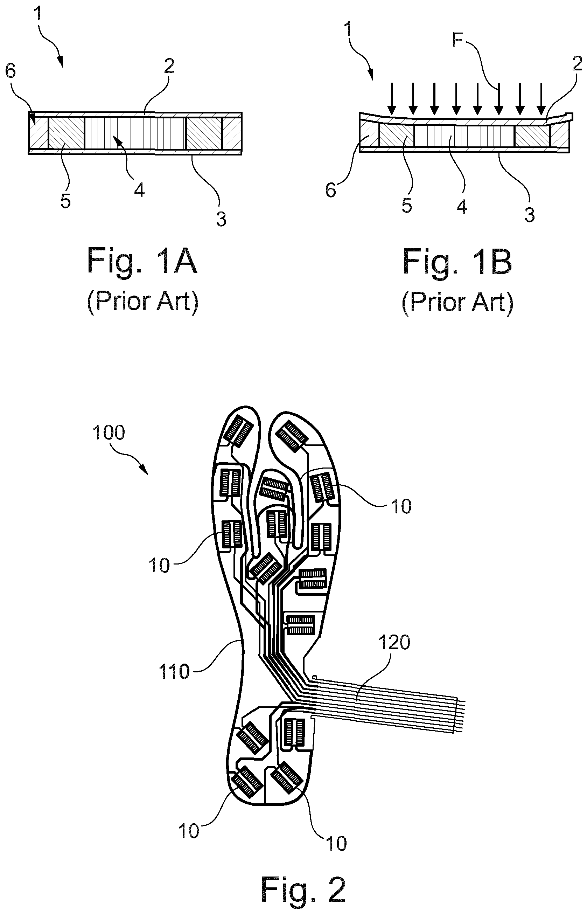

To better illustrate the above description of the FSR sensor, Figures 1A and 1B show in a schematic cross-sectional view the functional principle or principal constructive design, respectively, of the FSR sensor according to the prior art. Thereby, Figure 1A shows the FSR sensor 1 in an unloaded state and Figure 1B shows the FSR sensor 1 under the effect of force. The FSR sensor 1 comprises a first plastic/polymer membrane 2 and a second plastic/polymer membrane 3, which are separated from each other by an air gap 4. A semiconducting ink 5 is arranged between the first and second plastic/polymer membrane 2, 3 and extends over the entire length (or the thickness, respectively,) of the air gap 4. In addition, an adhesive layer 6 is arranged between the first and second plastic/polymer membrane 2, 3. In Figure 1B, the compression force F is applied on the surface of the first plastic/polymer membrane 2 in the thickness direction of the FSR sensor 1, causing mechanical deformation of the ink 5 as a result of compression. This results in a change in resistance due to the compression of the ink, which resistance - as described above - has a non-linear signal characteristic.

Due to the non-linear signal characteristics, the above mobile gait analysis systems are not well suited for use in mobile gait analysis because the inaccuracies of the measurement are too high. Further, shoes and insoles, for example, but especially human muscle and fat tissue, are all non-linear systems due to their material structures. The combination of several non-linear systems, some of which have unforeseen properties, poses a major challenge. In principle, non-linearity can lead to considerable deviations even with small deviations in the progressive range of the characteristic curve.

Therefore, there may be a need to reduce the impact of non-linear signal characteristics in mobile gait analysis systems.

US 2013/0213145 A1 describes sensor system that is adapted for use with an article of footwear and includes an insert member including a [inst layer and a second layer, a port connected to the insert and configured for communication with an electronic module, a plurality of force and/or pressure sensors on the insert member, Zllld a plurality of leads connecting the sensors to the port.

US 2014/0144251 A1 describes a film-type pressure sensor that includes a carrier structure including a first carrier film, a second carrier film and a spacer film arranged between the first and second carrier films, where one or more pressure-sensing cells are disposed in the carrier structure, each of which includes an electrode arrangement for producing an impedance change in response to a compressive force, an electrical interface is provided for mechanically and electrically connecting the pressure sensor to an evaluation circuit, Where some terminals of the interface are connected with the electrode arrangements of the cells so as to allow them to be read out, such that one or more electrical components interconnect at least two of the terminals in pairs so as to form a combination of terminal pair impedances that represents coded information relating to the pressure sensor.

US 2016/0287089 A1 describes a pressure sensor including hybrid electronic sheet and a wearable device including the pressure sensor. The pressure sensor has excellent controllable electric characteristics and excellent mechanical flexibility and stability, and measures, for example, pressure in a simple and highly reproducible manner in which a resistance of a component in a sensor varies depending on applied pressure.

### SUMMARY OF EMBODIMENTS

The present disclosure provides a sensor element, a sensor arrangement, a sensor system and a method, which reduces the impact of non-linear signal characteristics in mobile gait analysis systems.

A first aspect of the disclosure provides a sensor element that is particularly configured for detecting a force between a foot and a supporting surface. In particular, the sensor element may be configured to be arranged in a shoe or insole to perform the detection. The sensor element comprises:
a first layer comprising an electrically non-conductive carrier foil and a first semi-conductive layer extending at least partially along a longitudinal direction of the first layer and being in contact with the electrically conductive area,
a layer comprising a plurality of electrically conductive sections,
at least one electrical connection area, and
a second layer comprising an electrically non-conductive carrier foil, and a second semi-conductive layer extending at least partially along a longitudinal direction of the second layer,
wherein at least one electrical connection area is in contact with the one semi-conductive layer of the first and second layer and being adapted to be connected to an electric energy source, and
wherein the first semi-conductive layer and the second semi-conductive layer are arranged at a distance from one another and are adapted to selectively contact each other depending on the force applied to the sensor element, thereby changing a resistance value of the sensor element.

Thus, the sensor element may provide an at least quasi-linear or at least approximately linear signal characteristic in response to an applied force. The sensor element changes an electrical resistance due to changing (contact) surfaces as a result of the load or force applied. Further, the sensor element has a low input impedance in the lower single-digit kilo-ohm (kOhm) range. Preferably, the resistance value changes from a higher, first value to a lower, second value. The former may enable an exact determination of forces or the traceability of non-linear behavior to material properties of soles or human tissue, the latter may enable the use of simple electronic circuits for processing the signal. The sensor element may allow location-independent and selective load introduction, which is a helpful criterion for mobile gait analysis, as in a mobile setting in a flexible environment, such as in a shoe or insole, a constant and full-surface load introduction as required by an FSR sensor cannot be guaranteed. The non-linearity problems caused by the FSR technology have an essential origin in sensor mechanics as due to the low compression path of the used ink, material fluctuations as well as material changes due to environmental variations (temperature and humidity) are of considerable importance. In contrast to the FSR technology, the sensor element does not use a vertical path change (in relation to the thickness direction of the sensor element) but an at least essentially horizontal path change. Further, the semi-conductive layer does not have to have any compression properties, since it is not the semi-conductive layer that represents the measuring medium as such, but the contact surface. This allows a more homogeneous and clearly packed semi-conductive layer structure to be used. Since no compression is necessary, the semi-conductive layer structure can be made significantly flatter and minimalist. The above two points prevent the ageing of the ink or the alteration of its properties due to regular or excessive loading. This makes the sensor element virtually indestructible.

The sensor element may, at least in some embodiments, form part of a sensor matrix or, in at least some embodiments, be or constitute a sensor matrix. When the sensor element forms part of the sensor matrix, a plurality of sensor elements may be arranged adjacent to each other in a substantially common plane. Each sensor element may be configured to output an assigned signal that can change according to a load condition caused by the applied force. The carrier foil of the first and/or second layer may be formed from a suitable plastic, such as polyester or the like, and may have a thickness of about 50-150 microns (µm), preferably about 80-120 µm, preferably about 100 µm. The first and/or second semi-conductive layer may be formed as ink, in particular as carbon ink. The electrically conductive area may be formed by an electrically conductive ink, such as a silver-containing ink or silver ink, or the like. The electrically conductive area may serve as a connection to an electric circuit and may further serve as a conductor and connector of a carbon intermediate layer that may be arranged between the first and the second layer. This carbon layer may have a defined electrical resistance, which may be determined by the surface as well as by the mixture. The resistance of this surface may be chosen depending on the application. Silver has a higher electrical conductivity than graphite, for example, which significantly reduces the resistance, especially the input impedance.

According to an embodiment, the resistance value may be, preferably inversely, proportional to the surface area with which the two layers are in contact with each other. For example, the resistance value may decrease with increasing contact surface area. Thus, the sensor element is adapted to provide a linear signal characteristic.

In an embodiment, the resistance value may be at a maximum in an unloaded state. In other words, the resistance value may be at a maximum, when the contact surface area is at minimum or when the contact surface area is zero. More generally, the resistance value may decrease with increasing area size. This results in a low impedance output signal of the sensor element, which may be processed more easily, possibly also with lower-performance means, such as electronics.

According to an embodiment, the electrically conductive sections may reduce the overall resistance value of the first semi-conductive layer and/or of the sensor element. This may further improve signal characteristics and/or processing of the output signal of the sensor element.

In an embodiment, the resistance value may be reduced depending on the square measure of the stripe-shaped electrically conductive sections to which the vertical force is transferred.

According to an embodiment, the electrically conductive sections may be composed of silver ink, preferably applied, e.g. printed, onto an electrically non-conductive foil. The composition of the silver ink may be varied in order to, for example, adjust the signal characteristics or other characteristics of the sensor element.

In an embodiment, the electrically conductive sections, e.g. in form of a silver ink, may be applied with a thickness of about 0.5 to 15 µm, 1 to 10 µm, 2 to 8 µm, 3 to 6 µm, preferably about 5 µm. This low thickness results in a particularly flat sensor element.

According to an embodiment, the first semi-conductive layer may be composed of a carbon ink, preferably printed on top of the electrically conductive sections, and, optionally, thereby embedding the electrically conductive sections into the first semi-conductive layer.

In an embodiment, first semi-conductive layer may be applied with a thickness of about 1 to 30 µm, 2 to 20 µm, 5 to 15 µm, 8 to 12 µm, preferably about 10 µm. This low thickness results in a particularly flat sensor element.

According to an embodiment, the electrical connection area is composed of a silver ink. Optionally, it may be printed onto an electrically non-conductive foil.

In an embodiment, the electrical connection area may be applied with a thickness of about 0.5 to 15 µm, 1 to 10 µm, 2 to 8 µm, 3 to 6 µm, preferably about 5 µm. This low thickness results in a particularly flat sensor element.

According to an embodiment, the second semi-conductive layer may composed of a carbon-ink, preferably printed on top of the electrical connection area, and, optionally, thereby embedding the electrically conductive sections into the second semi-conductive layer.

In an embodiment, the second semi-conductive layer at a thickness of about 1 to 30 µm, 2 to 20 µm, 5 to 15 µm, 8 to 12 µm, preferably about 10 µm. This low thickness results in a particularly flat sensor element.

In an embodiment, the first semi-conductive layer and the second semi-conductive layer form two spaced-apart surfaces, which are spaced apart from each other in a unloaded state (when no force is applied to the sensor element) and in response to the force applied to the sensor element can be brought into contact, and a square measure, with which the two surfaces contact each other, is indicative for a resistance value of the sensor element. Thus, the sensor element changes an electrical resistance due to changing (contact) surfaces as a result of the load or force applied. This allows a contact area-oriented measurement, which results in an at least quasi-linear, approximately linear or linear sensor signal characteristic, since the effect of the size of the surfaces in contact can be defined proportionally to the resistance.

In an embodiment, the at least two first and/or second semi-conductive layers may be arranged at least partially parallel or non-parallel with respect to their longitudinal extension direction. As used herein, the overlapping area of the at least two first and/or second semi-conductive layers is determinative for the contact surface area of the sensor element.

In an embodiment, the at least two first and/or second semi-conductive layers may differ from each other in area. Preferably the at least two first and/or second semi-conductive layers differ from each other in area up to about 40 %, more preferably up to about 25 %, most preferably up to about 10%. In case when the at least two first and/or second semi-conductive layers differ from each other in area, the semi-conductive layer with the smaller area is determinative for the contact surface area of the sensor element.

In an embodiment, the at least two first and/or second semi-conductive layers may be arranged at least partially parallel with respect to their longitudinal extension direction and differ from each other in area. From a production point of view, it is of advantage that the said at least two first and/or second semi-conductive layers do not necessarily have to be arranged in a perfect parallel way and/or do not have the exact same area, because the overlapping area of the at least two first and/or second semi-conductive layers is determinative for the contact surface area of the sensor element. This gives room for production tolerances, which in turn reduces the production costs, especially as the first and the second layer of the sensor element with each of its components are fabricated as separate units first, before they are laminated together.

A plurality of electrically conductive sections is arranged on the carrier foil of the other one of the first and second layer and being in contact with the semi-conductive layer of the other one of the first and second layer, the electrically conductive sections extending transversely to the longitudinal direction of the other one of the first and second layer. The electrically conductive sections may also be referred to as bars or horizontal stripes. Further, the electrically conductive sections may be formed by an electrically conductive ink, such as a silver-containing ink or silver ink, or the like, and may have a thickness of about 1-20 µm, preferably about 2 to 10 µm, preferably about 5 µm. Thus, the electrically conductive sections may allow an uneven load on the sensor element, which is to be expected in at least the setting of shoe or an insole. The horizontal stripes not only allow the detection of signal variations in the longitudinal direction of the sensor element, as the arrangement of the stripes suggests (number of steps), but also in the transverse direction, since the resistance can also be modulated by stripes that are only partially resting on them. While the FSR technology requires a constant surface load, the sensor element, due to the plurality of electrically conductive sections, may be loaded at several points. It always delivers the same signal response.

The plurality of electrically conductive sections form, when considered or seen in a thickness direction of the sensor element, a keyboard-shape or claviature-shape. This allows for modelling the sensor element output signal in both the longitudinal direction and the transverse direction. In some embodiments, two or more keyboards or claviature may be provided, preferably arranged in one common plane.

The electrically conductive are stripe-shaped, and two adjacent strips are spaced apart in the longitudinal extension direction of the first and/or second layer. Thus, the sensor element may comprise an arrangement of cross stripes or horizontal stripes that works like a keyboard or clavier. A continuous semiconducting layer would react significantly differently here. In addition, the mechanical construction, particularly through the cross stripes or horizontal stripes, may allow not only signal variations in the longitudinal direction of the sensor to be detected, as the arrangement of the stripe suggests (number of steps), but also in the transverse axis, since the resistance may also be modelled by only partially exposed silver/carbon stripe.

In an embodiment, the plurality of electrically conductive sections may form or may be comprised by a layer. Further, in some embodiments, the layer and/or the electrically conductive sections may at least partially embedded in the semi-conductive layer of the first layer. This may affect and/or adjust the resistance, in particular reduce the resistance value and/or increase the electrical conductivity of the semi-conductive layer. Selective modification of the electrically conductive sections by, for example, modifying the dimensions thereof, the material composition, etc., may adjust the output signal characteristics of the sensor element.

According The sensor element according to any one of the preceding claims, wherein the electrical connection area, which preferably may be formed as a layer, may at least partially be embedded in the semi-conductive layer of the second layer.

In an embodiment, the electrically conductive sections may be configured to provide an electric signal that is at least substantially the same regardless of which individual section this is caused. Thus, when the load on the sensor element is distributed unequally, this load can also be recognized because the signal intensity, signal quality etc. is at least approximately the same in each case.

In an embodiment, the at least two first and/or second semi-conductive layers are arranged in parallel (or at least partially in parallel) with respect to their longitudinal extension direction, the two first and/or second semi-conductive layers are connected to each other via the at least one electrical connection area. Optionally, the at least two first and/or second semi-conductive layers may have a plurality of electrically conductive sections formed by an electrically conductive ink, the plurality of electrically conductive sections being arranged on the carrier foil of the other one of the first and second layer and being in contact with the semi-conductive layer of the other one of the first and second layer, the electrically conductive sections extending transversely to the longitudinal direction of the other one of the first and second layer. Thus, a sensitively measuring sensor element can be provided with a large number of measuring points in the longitudinal direction of the sensor and in the transverse direction of the sensor.

According to an embodiment, the first layer and the second layer may be laminated together with the first semi-conductive layer and the second semi-conductive layer spaced apart by a carbon intermediate layer and/or an adhesive arranged there between. In some embodiments, the laminate may comprise at least one peripheral contour having at least one notch that is directed inwards with respect to the peripheral contour and/or the laminate comprises at least one peripheral contour having at least one material recess that is arranged within the peripheral contour. Thus, a robust sensor element can be provided. If at least one notch and/or material recess is provided, formation of wrinkles in the foil or foils of the layers may at least be reduced or prevented, making the foil and/or the sensor arrangement more durable and robust.

In an embodiment, the first layer may be a bottom layer and the second layer may be a top layer. The terms "top" and "bottom" can refer, for example, to the arrangement in a shoe or insole, where the outsole is usually at the bottom and, when the shoe is worn or the insole is on the foot, the foot is on top.

A second aspect of the disclosure provides a sensor arrangement for detecting a force between a foot and a supporting surface. The sensor arrangement may be configured to be arranged in a shoe or insole to perform the detection. The sensor arrangement comprises:
a plurality of sensor elements according to any embodiment of the first aspect, the sensor elements arranged adjacent to each other in an at least substantially common plane,
a plurality of electrical lines, configured to connect the individual sensor elements to a first and second electrical potential and/or an evaluation device,
wherein the sensor arrangement comprises at least one peripheral contour having at least one notch that is directed inwards with respect to the peripheral contour and/or the sensor arrangement comprises at least one peripheral contour having at least one material recess that is arranged within the peripheral contour. Thus, formation of wrinkles in the foil or foils of the layers may at least be reduced or prevented, making the foil and/or the sensor arrangement more durable and robust. The shrink-wrapping or implementation of a sensor-equipped foil, particularly in an insole, may lead to the formation of wrinkles in the course of the load as a result of shearing movements of the upper and lower material of the insole. As a consequence of the formation of wrinkles, cracks may occur which affect both the electric lines and the sensors themselves. In addition to the shearing movement, the foil should also be able to withstand a 3D movement and deformation of the foot.

A third aspect of the disclosure provides a sensor system for detecting a force between a foot and a supporting surface. The sensor system may be configured to be arranged in a shoe or insole to perform the detection. The sensor system comprises:
at least one sensor element according to any embodiment of the first aspect, having a variable resistance and adapted to provide an electrical detection signal in response to the force applied thereto, and connected to a first electrical potential,
a resistor array, adapted to electrically influence the electrical detection signal of the sensor element, and connected to a second electrical potential,
a converter, having at least one input channel, adapted to obtain the electrically influenced electrical signal of the sensor element, and at least one output channel to provide a digital detection signal based on the converted electrical detection signal, and
a data processing means, adapted to determine a circuit configuration of one or more resistors of the resistor array used to influence the electrical detection signal.

Thus, the sensor system has a simple constructive design. Further, the threshold of the measuring range can thus be defined by selecting the resistors. It may also be possible to adjust the hardware to the expected load range. Further, a relative and sensor-specific reference point may be used, which makes it possible to make a calibration for only a measuring range. As long as the working range of the sensor element is in a linear part of its overall characteristic, this calibration may also be applicable to all sensor elements and works as long as the sensor element is not destroyed or disturbed by other influences. By using a relative reference point, disturbances due to changes in sensor dynamics (e.g. softer ink due to increased temperature or humidity) do not have any effect. Particularly for reasons of validity and reproducibility, not the entire voltage range is used, but only a range between about 45% and about 75% of the maximum voltage. If, for example, the resolution of the converter is 12 bit and thus has 4096 voltage levels, whereas other values, such as 8 bit, 16 bit etc. are also possible, this means that an under threshold is at 45% of the maximum voltage at voltage level 1843 and an upper threshold at 75% of the maximum voltage at voltage level 3072. Thus, 1229, namely 3072 - 1843 = 1229, voltage levels are available for the resolution or measurement of the force. If the sensor is designed for a force of 800 N/*cm*², for example, this means that a resolution of 0.65 N/bit can be achieved in an assumed linear range. By defining a lower and upper threshold, an approximately linear range may also be defined in an existing non-linear sensor characteristic.

In some embodiments, the resistor array, which may be a SIL resistor array, the converter, which may be an analog-to-digital converter (ADC) and/or the data processing means, which may be a suitable microprocessor, such as Arduino platform based microprocessors like e.g. Atmega328 or the like, may be arranged on a printed circuit board (PCB). It is noted that the resistor array may also be provided by internal pull-up resistors of the microprocessor, which resistors may switched on or off as required. Energy supply of the components may, for example, may be provided via an energy storage device, such as a battery, primary cell or secondary cell, or a power-generating element, such as a piezo element.

In some embodiments, analog inputs of the PCB may be connected via the resistor array to GND, for example, to (number of resistors of the resistor array) times (their resistance value), for example (8) x (2.2 kOhm) or other suitable values. These may be pull-down resistors that pull the AD inputs to zero when the input is empty. In some embodiments, the at least one sensor element having variable resistance values may be connected to the PCB and connected to the operating voltage, e.g. 3.3V or other suitable voltage values, on the opposite side, whereby the voltage at the converter increases proportionally to the drop in the external resistance. Assuming that the external resistors have a resistance value of 2.2KQ in the unloaded state, then the voltage at the converter may be 50% of the operating voltage.

According to an embodiment, the data processing means may be further adapted to determine the circuit configuration of the one or more resistors related to a relative resistance or relative voltage value.

Thus, defining the threshold of the measuring range by selecting the resistors may be further improved. If a relative resistance or a relative voltage is used as reference point, an offset remains at 50% (e.g. 2048 bit) and the measuring range remains constant regardless of the respective sensitivity.

In an embodiment, the data processing means may be further adapted to, by determining the circuit configuration of the one or more resistors to be used, setting a measuring range of the converter to be used, which measuring range is smaller than a predetermined resolution of the converter. Thus, defining the threshold of the measuring range by selecting the resistors may be further improved.

According to an embodiment, the data processing means may be further adapted to determine a characteristic of the detection signal and to set the measuring range between an upper limit and a lower limit, and wherein the characteristic of the detection signal between the upper limit and the lower limit is determined to be at least quasi-linear. Thus, defining the threshold of the measuring range by selecting the resistors may be further improved.

In an embodiment, the data processing means may be further adapted to set the upper limit and/or the lower limit of the measuring range related to a desired sensitivity of the sensor element. Thus, defining the threshold of the measuring range by selecting the resistors may be further improved.

A fourth aspect of the disclosure provides a method for detecting a force between a foot and a supporting surface. The method may be particularly suitable for use with the sensor system according to the second aspect. The method comprises:
obtaining a force detection signal,
analyzing the force detection signal to detect at least a non-linear characteristic of the detection signal,
generating a configuration signal, comprising a configuration of one or more resistors of a resistor array which are to be used to influence an electrical detection signal on which the force detection signal is based, wherein the configuration is determined on basis of the detected non-linear characteristic, and
providing the configuration signal.

Thus, the threshold of the measuring range can be defined by selecting the resistors used. It may also be possible to adjust the hardware to the expected load range. Further, a relative and sensor-specific reference point may be used, which makes it possible to make a calibration for only a measuring range. By using a relative reference point, disturbances due to changes in sensor dynamics (e.g. softer ink due to increased temperature or humidity) do not have any effect. Particularly for reasons of validity and reproducibility, not the entire voltage range is used, but only a range between about 45% and about 75% of the maximum voltage. Such a window-based approach, i.e. limiting the measuring range, also allows minor adjustments to be made with regard to sensitivity without having to resort to another sensor. It could also make a sensor in the lower voltage range less sensitive or in the upper range more sensitive, depending on how large the range is or where the limit ranges lie on the curve.

In some embodiments, the force detection signal may be obtained by at least one sensor element according to any embodiment of the first aspect.

According to an embodiment, after providing the configuration signal, the configuration of the resistance array may be set.

In an embodiment, after providing the configuration signal, a measurement operation of a sensor system may be performed using at least one sensor element connected to the resistance array and the configuration of the resistance array defines a measurement range of the at least one sensor element having a lower and an upper threshold. Thus, the threshold of the measuring range can be defined by selecting the resistors used. It may also be possible to adjust the hardware to the expected load range. Further, a relative and sensor-specific reference point may be used, which makes it possible to make a calibration for only a measuring range. By using a relative reference point, disturbances due to changes in sensor dynamics (e.g. softer ink due to increased temperature or humidity) do not have any effect. Particularly for reasons of validity and reproducibility, not the entire voltage range is used, but only a range between about 45% and about 75% of the maximum voltage. Such a window-based approach, i.e. limiting the measuring range, also allows minor adjustments to be made with regard to sensitivity without having to resort to another sensor. It could also make a sensor in the lower voltage range less sensitive or in the upper range more sensitive, depending on how large the range is or where the limit ranges lie on the curve.

A fifth aspect of the disclosure provides a computer-implemented method for analyzing and/or diagnosing the gait of a subject. The method comprises the steps of
determining a time course of a ground reaction force of at least one gait cycle of a subject, using a sensor system according to the third aspect,
normalizing the determined time course of the ground reaction force,
comparing the normalized time course with a reference, and
determining the gait of the subject based on a result of said comparison.

In other words, the sensor system according to the third aspect may be used to diagnose, monitor and/or analyze the gait of a subject. For this purpose one or more of the sensor elements of the first aspect may be arranged, preferably as a sensor arrangement or the like, in a shoe, insole, etc. The step of normalizing the determined time course and/or comparing the normalized time course with a reference, and/or determining the gait of the subject based on a result of said comparison may be carried out automatically by the sensor system itself by, for example, using the data processing means of the third aspect, or by a separate computer device, such as a personal computer, a smartphone, etc., which may also be arranged remote to the sensor system, such as a computing cloud, a server or the like. The reference may be a dataset, stored e.g. in a database or the like. The step of comparing the normalized time course with a reference and/or determined the gait may utilize a numerical method, an estimation method, or the like.

A sixth aspect of the disclosure, which is not covered by the claims, provides a computer program for detecting a force between a foot and a supporting surface, comprising instructions which, when the program is executed by a computer and/or data processing means, e.g. the data processing means of the third aspect, cause the computer to carry out the method according to any embodiment of the fourth aspect. As used herein, the force between a foot and a supporting surface of the above aspects may also be referred to as the ground reaction force.

A seventh aspect of the disclosure, which is not covered by the claims, provides a computer-readable medium having stored the computer program of the sixth aspect.

A computer program element might therefore be stored on a computer unit, which might also be an embodiment of the present disclosure. This computing unit may be adapted to perform or induce performance of the steps of the method described above. Moreover, it may be adapted to operate the components of the above-described apparatus. The computing unit can be adapted to operate automatically and/or to execute orders of a user. A computer program may be loaded into a working memory of a data processor. The data processor may thus be equipped to carry out the method of the disclosure. This exemplary embodiment of the disclosure covers both the computer program that has the intervention installed from the beginning, and a computer program that by means of an update turns an existing program into a program that uses the disclosure. A computer program may be stored and/or distributed on a suitable medium, such as optical storage media, or a solid state medium supplied together with, or as a part of other hardware, but may also be distributed in other forms, such as via the Internet or other wired or wireless telecommunication systems. However, the computer program may also be presented over a network like the World Wide Web, and can also be downloaded into the working memory of a data processor from such a network.

It should be noted that embodiments as described above may be combined with respect to each other so as to gain a synergetic effect, which may extend over the separate technical effects of the single features. Exemplary embodiments of the present disclosure will be described in the following. Further, embodiments of the disclosure are described with reference to different subject-matters. In particular, some embodiments are described with reference to method-type claims, whereas other embodiments are described with reference to device-type claims. However, a person skilled in the art will gather from the above, and the following description that, unless otherwise notified, in addition to any combination of features belonging to one type of subject-matter, also other combinations between features relating to different subject-matters is considered to be disclosed with this application.

### BRIEF DESCRIPTION OF THE DRAWINGS

Exemplary embodiments of the disclosure will be described in the following with reference to the following drawings.
- Figure 1A: shows in a schematic sectional view a FSR (Force Sensing Resistor) sensor in an unloaded state, according to the prior art.
- Figure 1B: shows in a schematic sectional view the FSR sensor of Figure 1A according to the prior art under the effect of force.
- Figure 2: shows a top view of a sensor arrangement for detecting a force between a foot and a supporting surface according to an embodiment of the disclosure.
- Figure 3: shows a top view of a sensor arrangement for detecting a force between a foot and a supporting surface according to an embodiment of the disclosure.
- Figure 4: shows an exemplary application of a sensor arrangement according to an embodiment of the disclosure in a sole compound.
- Figure 5: shows in an exploded view a constructive structure of a sensor element for detecting a force between a foot and a supporting surface according to an embodiment of the disclosure.
- Figure 6A: shows in a schematic sectional view a sensor element according to an embodiment of the disclosure in an unloaded state.
- Figure 6B: shows in a schematic sectional view a sensor element under the effect of force.
- Figures 7A to 7D: show in a schematic top view different load states of a sensor element according to an embodiment of the disclosure.
- Figure 8: shows in a schematic block diagram a sensor system for detecting a force between a foot and a supporting surface according to an embodiment of the disclosure.
- Figure 9: shows in a flow chart a method for detecting a force between a foot and a supporting surface according to an embodiment of the disclosure.
- Figure 10: shows in a flow chart a computer-implemented method for diagnosing and/or monitoring and/or analyzing the gait of a subject according to an embodiment of the disclosure.
- Figure 11: shows in a force-voltage/resistance diagram a signal characteristic which is linearized by a method and/or a system according to an embodiment of the disclosure.
- Figure 12: shows a graphical representation of an exemplary result of a computer-implemented method for analyzing the gait of a subject according to an embodiment of the disclosure.
- Figures 13A to 13E: show in a schematic top view alternative arrangements of a layer comprising a plurality of electrically conductive sections according to an embodiment of the disclosure.
- Figure 14: shows alternative arrangements of first and second semi-conductive layers to each other according to an embodiment of the disclosure.

### DETAILED DESCRIPTION OF EXEMPLARY EMBODIMENTS

In the following, a detailed description of exemplary embodiments will be given to explain the disclosure in more detail.

Figure 2 shows an exemplary embodiment of a sensor arrangement 100 for detecting a force between a foot and a supporting surface, which allows a gait analysis. The sensor arrangement 100 is configured to be arranged in a shoe or insole to perform the detection and comprises a plurality of sensor elements 10 arranged adjacent to each other in an at least substantially common plane which here is formed by a carrier 110. It should be noted that although only four sensor elements are identified by reference signs, the number of sensor elements may differ from four, as shown in Figure 2, where a total of 15 sensor elements distributed over the foot root, metatarsus and toes are shown as examples. Further, the sensor arrangement 100 comprises a plurality of electrical lines 120, configured to connect the individual sensor elements to further parts of the sensor arrangement 100 and/or a sensor system 200 that will be described below. The electrical lines 120 may be printed, for example, or formed by cables with a small cross section, etc.

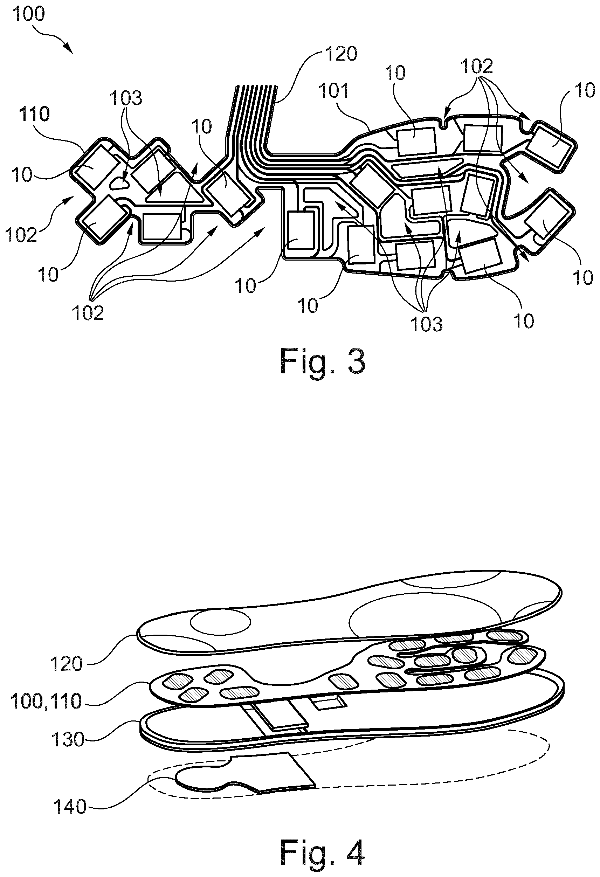

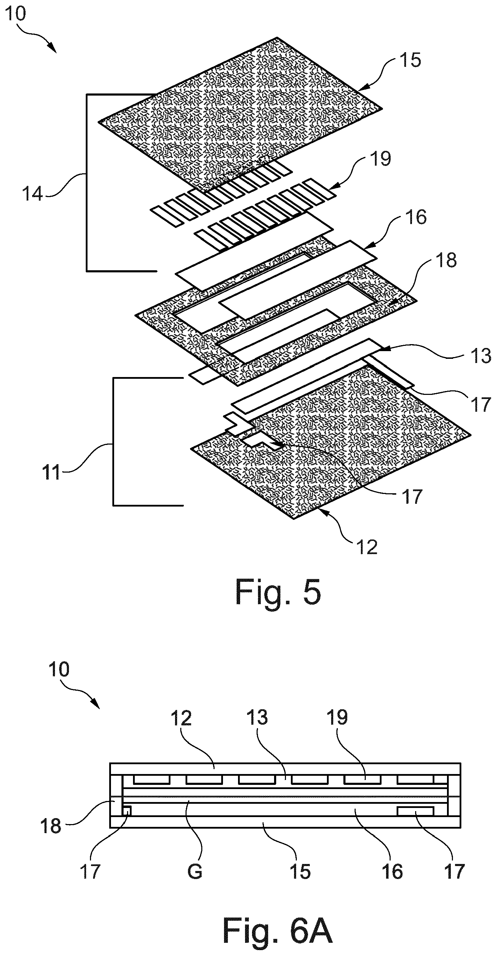

Figure 3 shows a further exemplary embodiment of the sensor arrangement 100 for detecting a force between a foot and a supporting surface. As can be seen in Figure 3, at least in some embodiments, the sensor arrangement 100 comprises at least one peripheral contour 101 having at least one notch 102 that is directed inwards with respect to the peripheral contour 101, which may also be referred to as a circumferential contour that encloses the common plane in which the sensor elements 10 are arranged. In addition or alternatively to the at least one notch 102, the sensor arrangement 100 comprises at least one material recess 103 that is arranged within the peripheral contour 101. If the sensor arrangement 100 is multilayered, the material recess 103 may refer to at least some of the layers (e.g. a top layer, a bottom layer and/or an intermediate layer as described below, see also Figure5) or, preferably, to all layers, so that the material recess extends over the entire thickness direction of the sensor arrangement 100.

Figure 4 shows an exemplary embodiment of a sole compound, which represents an exemplary application for the sensor arrangement 100. The sole compound is arranged in the thickness direction of the sensor arrangement 100 and comprises the sensor arrangement 100, which is arranged in a sandwich arrangement between a cover sole 120, a bottom sole 130, and an optional battery protection 140. The sensor arrangement 100 is arranged particularly between the cover sole 120 and the bottom sole 130. The sensor arrangement 100 may also be adapted to different purposes by a variation of the sole compound.

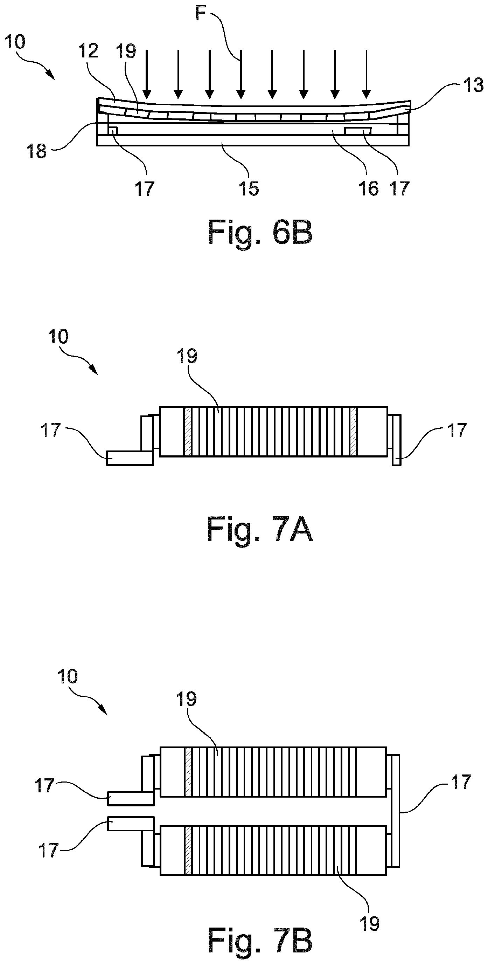

Figure 5 shows in an exploded view a constructive structure of an exemplary one of the sensor elements 10 according to an exemplary embodiment. Usually, the sensor elements 10 of the sensor arrangement 110 are identical to each other. The sensor element 10 comprises a first layer 11 comprising an electrically non-conductive carrier foil 12 or film and a first semi-conductive layer 13 extending at least partially along a longitudinal direction of the first layer 11. The sensor element 10 further comprises a second layer 14 comprising an electrically non-conductive carrier foil 15 or film, and a second semi-conductive layer 16 extending at least partially along a longitudinal direction of the second layer 14. By way of example, the first layer 11 is a bottom layer and the second layer is a top layer, wherein a foot would be placed from the top layer. At least one electrical connection area 17 is in contact with the one semi-conductive layer of the first and second layer 11, 14 and is adapted to be connected to an electric energy source (not shown). By way of example, the first and/or second semi-conductive layer 16 are formed by a carbon ink. In at least some embodiments, the first layer 11 and the second layer 14 are laminated together with the first semi-conductive layer 13 and the second semi-conductive layer spaced 16 apart by an intermediate carbon layer and/or an adhesive layer 18 arranged there between. By way of example, as can be seen in Figure 5, the electrical connection area 17 is in contact with the first semi-conductive layer 13 of the first layer 11 of the sensor element 10. It is noted that the first semi-conductive layer 13 and the second semi-conductive layer 16 are arranged at a distance from one another and are adapted to selectively contact each other depending on a force applied to the sensor element 10, as this can be seen in e.g. Figures 6A and 6B.

Still referring to Figure 5, at least in some embodiments, the first semi-conductive layer 13 and the second semi-conductive layer 16 form two spaced-apart surfaces, which are spaced apart from each other in a unloaded state and in response to the force applied to the sensor element 10 can be brought into contact, and a square measure, with which the two surfaces contact each other, is indicative for a resistance value of the sensor element 10 (see also Figures 6A and 6B for more details). It is noted that the square measure may also be referred to as the area that can be specified in square millimeters (*mm*²) or the like, for example, with which the first semi-conductive layer 13 and the second semi-conductive layer 16 contact each other.

Further, at least in some embodiments, at least two first and/or second semi-conductive layers 13, 16 are arranged in parallel with respect to their longitudinal extension direction, the two first and/or second semi-conductive layers 13, 16 are connected to each other via the at least one electrical connection area 17. This can be seen in Figure 5, where two layers are arranged next to each other. It is noted that a further electrical connections area 17 is arranged on an opposite side to the other electrical connection area 17.

Still referring to Figure 5, at least in some embodiments, a plurality of electrically conductive sections 19 is arranged on the carrier foil 12, 15 of the other one of the first and second layer 11, 14 and being in contact with the semi-conductive layer 13, 16 of the other one of the first and second layer 11, 14, the electrically conductive sections 19 extending transversely to the longitudinal direction of the other one of the first and second layer 11, 14. By way of example, as can be seen in Figure 5, the electrically conductive sections 19 are arranged on the carrier foil 15 of the second layer 14. The electrically conductive sections 19 are arranged between the carrier foil 15 and the second semi-conductive layer 16. Further, the electrically conductive sections 19 of the second layer are stripe-shaped, and two adjacent strips are spaced apart in the longitudinal extension direction of the first and/or second layer 11, 14. By way of example, the electrically conductive sections 19 are formed by or comprise a silver ink.

Referring to Figures 6A and 6B, the functional principle of the sensor element 10 is explained in the following. Figure 6A shows the sensor element 10 in an unloaded state and Figure 6B shows the sensor element 10 under the effect of force, which force may also be referred to as a ground reaction force as a parameter in gait analysis. As can be seen in Figure 6A, in the unloaded state, the surfaces of the first and second semi-conductive layer 13, 16 of the first and second layer 11, 14 are at least slightly spaced apart from each other and are therefore not in contact with each other, or in only slightly or not electrically conductive contact with each other. Optionally, there may be e.g. an air gap G between the two semi-conductive layers 13, 16. In this unloaded state, the sensor element 10 thus has a first resistance value that roughly corresponds to the input impedance. In Figure 6B, a compression force F is applied on the outer surface of the second layer 14 in the thickness direction of the sensor element 10, causing mechanical deformation at least the second layer 14 as a result of compression. The mechanical deformation causes the two surfaces of the first and second semi-conductive layers 13, 16 to come closer to each other, at least in sections, or, respectively, a reduction in the size of the air gap G, at least in sections. In other words, this causes the second semi-conductive layer 16 to be pressed in the direction of the first semi-conductive layer 13 of the first layer 11. Depending on the size of the surface contact, i.e. the square measure, between the first and second semi-conductive layer 13, 16, the resistance of the sensor element 10 changes depending on the force F applied thereto. In principle, a horizontal path change is used here, in contrast to an FSR sensor (see Figures 1A and 1B showing the prior art). By changing the measuring principle from vertical (as used at FSR sensors) to horizontal (as used herein), the path changes from a few µm to several mm to even several centimeters. As a result, possible material fluctuations and environmental effects on the ink structure are hardly noticeable. By way of example, for a sensor dimension of 16.7 mm for the sensor element 10 herein, and approximately 30 nm for FSR sensors are compared. This corresponds to a ratio of approximately 5000:1. Material defects thus affect the sensor signal by less than one 5000th - the same with regard to material fluctuations due to changing environmental conditions. Another effect achieved by a surface-oriented measurement is the creation of a linear sensor signal characteristic, since the effect of the area can be defined in proportion to the resistance. Due to the more compact and homogeneous structure of the carbon ink, smaller screens can also be used in production, resulting in a much more precise print image. This enables production tolerances of less than 1%. The more homogeneous surface of the ink may also allow better or more defined over printability (application of several layers).

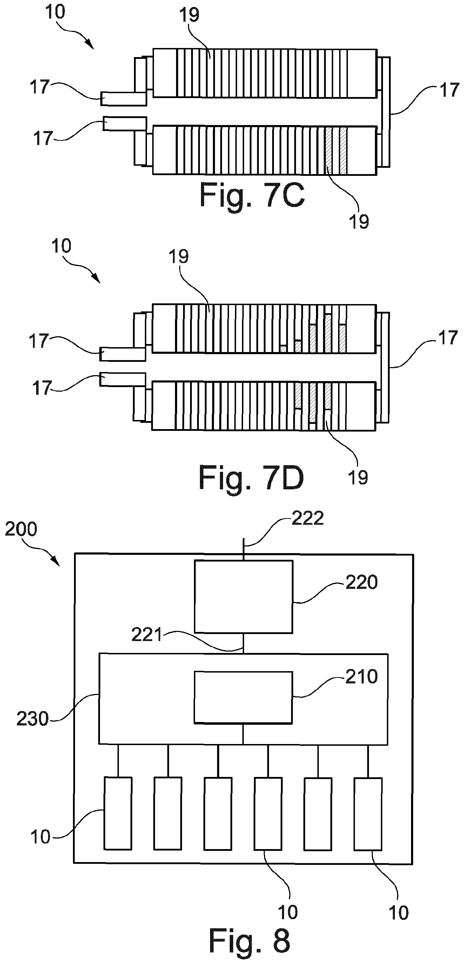

Figures 7A to 7D show in a schematic top view different load states of the sensor element 10 according to an embodiment. The different load states of the sensor element 10 are indicated in Figures 7A to 7D by different or differently arranged hatchings, which can be seen at least by comparing the individual Figures with each other. By way of example, the sensor element 10 comprises two first and/or second semi-conductive layers 13, 16 that are arranged in parallel with respect to their longitudinal extension direction and/or arranged in a common plane, the two first and/or second semi-conductive layers 13, 16 are connected to each other via the at least one electrical connection area 17. Further electrical connection areas form 17 are provided for direct or indirect connection to electrical potentials or a resistor array. In some embodiments, only part, e.g. half, of the above arrangement may be provided, as indicated in Figure 7A. In Figures 7A to 7 D, it can be seen that the stripe-shaped electrically conductive sections 19 form a kind of keyboard or claviature. The stripe-shaped electrically conductive sections 19 serve, for example, to reduce the electrical resistance of the semi-conductive layers 13, 16. The number and/or thickness of the strips may be varied to regulate the drop in electrical resistance and/or signal sensitivity. The response behavior of the sensor may also be modelled by adjusting the width of the electrically conductive sections 19 and/or the properties, e.g. material properties, of the semi-conductive layers 13, 16. The stripe-shaped electrically conductive sections 19 may also allow an uneven load on the sensor element 10, which is to be expected when used in an insole. While the FSR technology (see Figures 1A and 1B showing the prior art) requires a constant surface load, the sensor element 10 herein may be loaded at several points. It always delivers the same signal response.

Figure 8 shows in a schematic block diagram a sensor system 200 for detecting a force between a foot and a supporting surface. The sensor system 200 comprises, for example, a plurality of the sensor elements 10 as described above. These have a variable resistance and adapted to provide an electrical detection signal in response to the force applied thereto, and connected to a first electrical potential (not shown). Further, the sensor system 200 comprises a resistor array 210, adapted to electrically influence the electrical detection signal of the sensor element 10, and connected to a second electrical potential (not shown). Further, the sensor system 200 comprises a converter 220, having at least one input channel 221, adapted to obtain the electrically influenced electrical signal of the sensor element 10, and at least one output channel 222 to provide a digital detection signal based on the converted electrical detection signal. Further, the sensor system 200 comprises a data processing means 230, adapted to determine a circuit configuration of one or more resistors of the resistor array 210 used to influence the electrical detection signal. By way of example, the resistor array 210 is here part of the data processing means 230, which is a microprocessor. It should be noted that the data processing means 230 and the resistor array 210 may also be separate, although this is not explicitly shown here.

In at least some embodiments, the data processing means 230 is further adapted to determine the circuit configuration of the one or more resistors related to a relative resistance or voltage value.

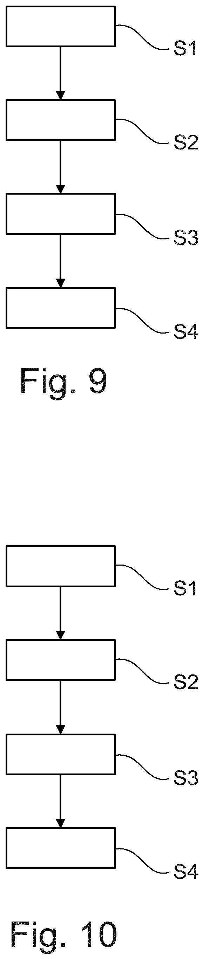

In at least some embodiments, the data processing means 230 is further adapted to, by determining the circuit configuration of the one or more resistors to be used, setting a measuring range of the converter 220 to be used, which measuring range is smaller than a predetermined resolution of the converter 220. In at least some embodiments, the data processing means 230 is further adapted to determine a characteristic of the detection signal and to set the measuring range between an upper limit and a lower limit, and wherein the characteristic of the detection signal between the upper limit and the lower limit is determined to be at least quasi-linear (see e.g. Figure 10). In at least some embodiments, the data processing means 230 is further adapted to set the upper limit and/or the lower limit of the measuring range related to a desired sensitivity of the sensor element 10.

Figure 9 shows in a flow chart a method for detecting a force between a foot and a supporting surface. In a step S1, a force detection signal is obtained, for example, by at least of the sensor elements 10 as described above. In a step S2, the force detection signal in analyzed to detect at least a non-linear characteristic of the detection signal. In as step S3, a configuration signal is generated, the configuration signal comprising a configuration of one or more resistors of the resistor array 220 which are to be used to influence an electrical detection signal on which the force detection signal is based, wherein the configuration is determined on basis of the detected non-linear characteristic. In a step S4, the configuration signal is provided, for example, to the processing means 230.

Figure 10 shows in a flow chart a computer-implemented method for diagnosing and/or monitoring and/or analyzing the gait of a subject, for which method the above system 200 can be used. In a first step S 1, a time course of a ground reaction force of at least one gait cycle of a subject is determined. In a step S2, the determined time course of the ground reaction force is normalized. In a step S3, the normalized time course is compared with a reference. Then, in a step S4, the gait of the subject is determined based on a result of said comparison.

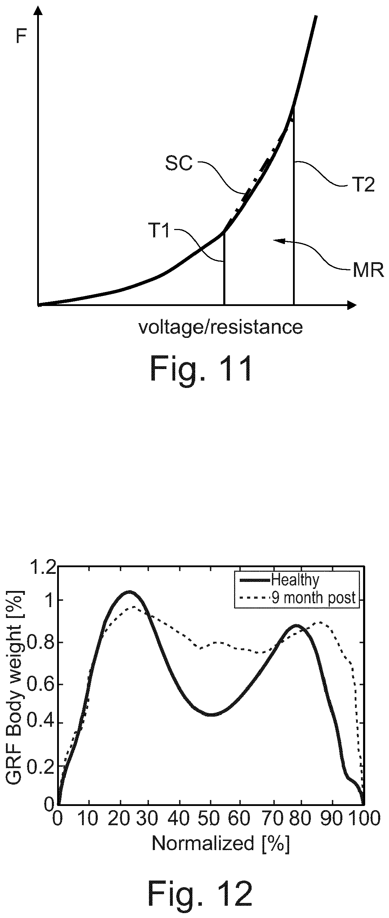

Figure 11 shows in a force-voltage/resistance diagram a signal characteristic SC of the sensor element 10 which is linearized by the above sensor system 200 and/or the above method. As can be seen, the threshold of a measuring range MR of the sensor element 10 can be defined or set by selecting the resistors used. By way of example, the measuring range MR is set between a lower threshold T1 and an upper threshold T2, resulting in a window-based approach. In the set measuring range MR, the sensor element signal characteristic SC of the sensor element 10 is quasi-linear, as indicated by a dashed line.

Figure 12 shows a graphical representation of an exemplary result of the above computer-implemented method for analyzing the gait of a subject using the sensor system (200). Herein, the time course of the ground reaction force of one gait cycle of a diseased subject (surgical intervention of the leg) was determined, normalized to the body weight of the subject and to the contact time of the foot, and compared the normalized time course of a reference (healthy subject). Herein, the dotted line represents the normalized time course of a subject after a surgical intervention of the leg, while the solid line represents the reference, namely the time course of a ground reaction force of one gait cycle of a healthy subject. Visual comparison of the dotted line to the solid line clearly shows an improvement of the gait cycle of the subject after 9 months after the surgical intervention, but still a difference to the reference (healthy subject).

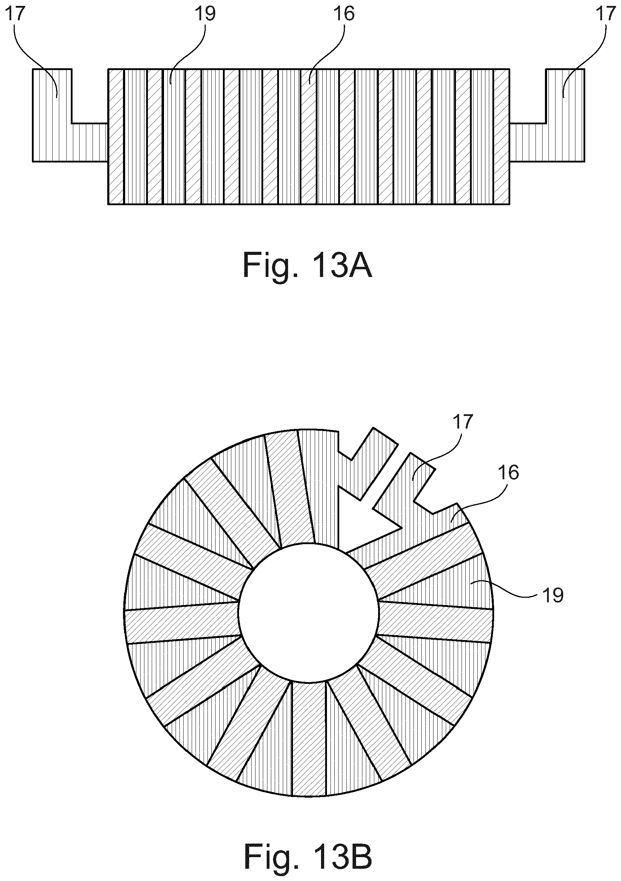

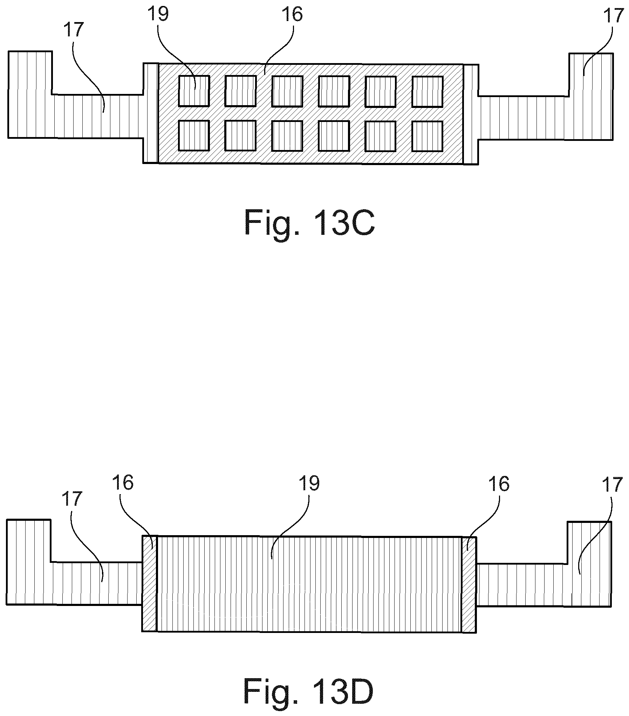

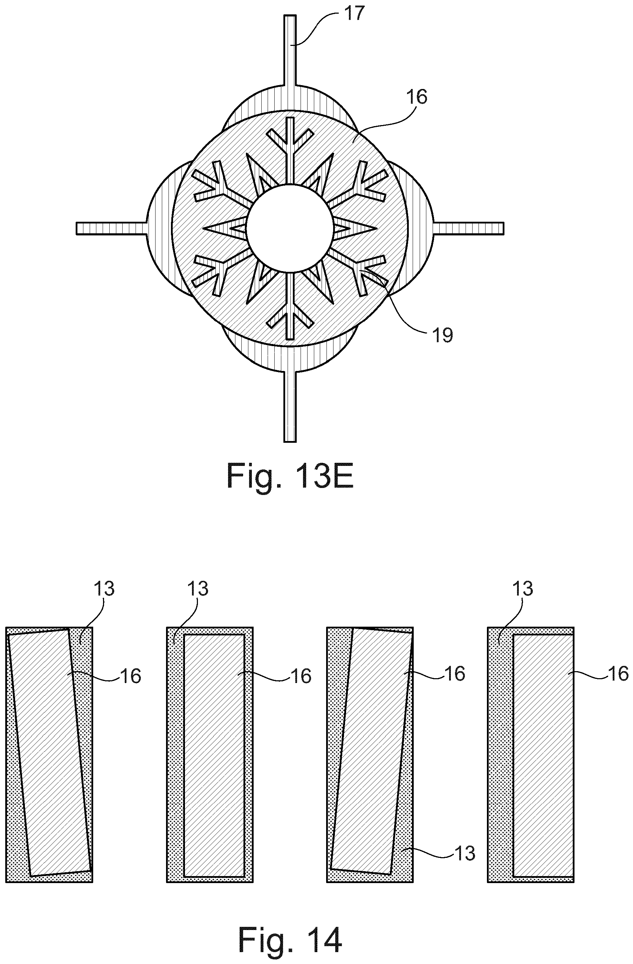

Figures 13A to 13E show alternative arrangement of the layer comprising a plurality of electrically conductive sections 19, The light-colored longitudinal striped areas represent the electrically conductive sections 19, e.g. formed by or comprising a silver ink, while the dark-colored diagonally striped areas represent the semi-conductive layer 16. The alternative arrangements comprise the electrically conductive sections 19 in form of striped patterns (Fig 12A), triangular patterns (Figure 12B), rectangular patterns (Figure 12C), single area patterns (Figure 12D) or dendritic patterns (Fig. 12E).

Figure 14 shows alternative arrangements of the first and second semi-conductive layers 13, 16 to each other. Herein, the second semi-conductive layer 16, which is part of the second layer 14, is smaller in size, when compared to the first semi-conductive layer 13, which is part of the first layer 11. Notably, the contact surface area of the sensor element is determined by the overlapping area of the first and second semi-conductive layers 13, 16.

## Claims

1. A sensor element (10) for detecting a force between a foot and a supporting surface, comprising:
a first layer (11) comprising an electrically non-conductive carrier foil (12) and a first semi-conductive layer (13) extending at least partially along a longitudinal direction of the first layer (11),
a second layer (14) comprising an electrically non-conductive carrier foil (15), and a second semi-conductive layer (16) extending at least partially along a longitudinal direction of the second layer (14),
a layer comprising a plurality of electrically conductive sections (19), wherein the plurality of electrically conductive sections (19) form, when considered in a thickness direction of the sensor element, a keyboard-shape or claviature-shape with the electrically conductive sections (19) being stripe-shaped, and two adjacent strips being spaced apart in the longitudinal extension direction of the second layer (14),
at least one electrical connection area (17), and
wherein the at least one electrical connection area (17) is in contact with the one semi-conductive layer (13, 16) of the first and second layer (14) and being adapted to be connected to an electric energy source, and
wherein the first semi-conductive layer (13) and the second semi-conductive layer (16) are arranged at a distance from one another and are adapted to selectively contact each other depending on the force applied to the sensor element (10), thereby changing a resistance value of the sensor element (10).

2. The sensor element according to claim 1, wherein the first semi-conductive layer (13) and the second semi-conductive layer (16) form two surfaces facing each other, which are spaced apart from each other in a unloaded state and, in response to the force applied to the sensor element (10), can be brought into contact, and wherein a square measure, with which the two surfaces contact each other, is indicative for the resistance value of the sensor element (10).

3. The sensor element according to any one of the preceding claims, wherein the plurality of electrically conductive sections (19) is arranged on the carrier foil (, 15) of the second layer (14) and being in contact with the semi-conductive layer (16) of the second layer (, 14), the electrically conductive sections extending transversely to the longitudinal direction of the second layer (, 14).

4. The sensor element according to any one of the preceding claims, wherein a layer comprising the plurality of electrically conductive sections (19) is at least partially embedded in the semi-conductive layer (16) of the second layer (14).

5. The sensor element according to any one of the preceding claims, wherein the electrical connection area (17), which preferably is formed as a layer, is at least partially embedded in the semi-conductive layer (13) of the first layer (11).

6. The sensor element according to any one of the preceding claims, wherein at least two first and/or second semi-conductive layers (13, 16) are arranged in parallel with respect to their longitudinal extension direction, the two first and/or second semi-conductive layers (13, 16) are connected to each other via the at least one electrical connection area (17).

7. The sensor element according to any one of the preceding claims, wherein the first layer and the second layer (11, 14) are laminated together with the first semi-conductive layer and the second semi-conductive layer spaced apart by an intermediate carbon layer and/or an adhesive (18) arranged there between.

8. A sensor arrangement (100) for detecting a force between a foot and a supporting surface, comprising:
a plurality of sensor elements (10) according to any one of the preceding claims,
the sensor elements (10) arranged adjacent to each other in an at least substantially common plane,
a plurality of electrical lines (120), configured to connect the individual sensor elements to a first and second electrical potential and/or an evaluation device,
wherein the sensor arrangement (100) comprises at least one peripheral contour (101) having at least one notch (102) that is directed inwards with respect to the peripheral contour (101) and/or the sensor arrangement (100) comprises at least one peripheral contour (101) having at least one material recess (103) that is arranged within the peripheral contour (101).

9. A sensor system (200) for detecting a force between a foot and a supporting surface, comprising:
at least one sensor element (10) according to any one of claims 1 to 7, having a variable resistance and adapted to provide an electrical detection signal in response to the force applied thereto, and connected to a first electrical potential,
a resistor array (210), adapted to electrically influence the electrical detection signal of the sensor element, and connected to a second electrical potential,
a converter (220), having at least one input channel, adapted to obtain the electrically influenced electrical signal of the sensor element, and at least one output channel to provide a digital detection signal based on the converted electrical detection signal, and
a data processing means (230), adapted to determine a circuit configuration of one or more resistors of the resistor array used to influence the electrical detection signal.

10. The sensor system according to claim 9, wherein the data processing means (230) is further adapted to determine the circuit configuration of the one or more resistors related to a relative resistance or voltage value.

11. The sensor system according to claim 9 or 10, wherein the data processing means (230) is further adapted to, by determining the circuit configuration of the one or more resistors to be used, setting a measuring range of the converter to be used, which measuring range is smaller than a predetermined resolution of the converter (220).

12. The sensor system according to claim 11, wherein the data processing means (230) is further adapted to determine a characteristic of the detection signal and to set the measuring range (MR) between an upper limit (T2) and a lower limit (T1), and wherein the characteristic (SC) of the detection signal between the upper limit (T2) and the lower limit (T1) is determined to be at least quasi-linear.

13. The sensor system according to claim 12, wherein the data processing means is further adapted to set the upper limit and/or the lower limit of the measuring range (MR) related to a desired sensitivity of the sensor element (10).

14. A method for detecting a force between a foot and a supporting surface, by using a sensor system (200) according to any one of claims 9 to 13, the method comprising:
obtaining, from a sensor element (10), a force detection signal,
analyzing the force detection signal to detect at least a non-linear characteristic of the detection signal,
generating a configuration signal, comprising a configuration of one or more resistors of a resistor array which are to be used to influence an electrical detection signal on which the force detection signal is based, wherein the configuration is determined on basis of the detected non-linear characteristic, and
providing the configuration signal, wherein the configuration signal is to be used to set the configuration of the resistance array (210).

15. A computer-implemented method for analyzing the gait of a subject, comprising:
determining a time course of a ground reaction force of at least one gait cycle of a subject, using a sensor system (200) according to any one of claims 9 to 13,
normalizing the determined time course of the ground reaction force,
comparing the normalized time course with a reference, and
determining the gait of the subject based on a result of said comparison.

## Patentansprüche

1. Ein Sensorelement (10) zum Detektieren einer Kraft zwischen einem Fuß und einer stützenden Oberfläche, aufweisend:
eine erste Schicht (11), welche eine elektrisch nicht leitende Trägerfolie (12) und eine erste halbleitende Schicht (13) aufweist, welche sich zumindest teilweise entlang einer longitudinalen Richtung der ersten Schicht (11) erstreckt,
eine zweite Schicht (14), welche eine elektrisch nicht leitende Trägerfolie (15) und eine zweite halbleitende Schicht (16) aufweist, welche sich zumindest teilweise entlang einer longitudinalen Richtung der zweiten Schicht (14) erstreckt,
eine Schicht, welche eine Mehrzahl von elektrisch leitenden Abschnitten (19) aufweist, wobei die Mehrzahl von elektrisch leitenden Abschnitten (19) in einer Dickenrichtung des Sensorelements betrachtet eine Tastaturform oder eine Klaviaturform ausbildet, wobei die elektrisch leitenden Abschnitte (19) streifenförmig sind und zwei benachbarte Streifen sind in der longitudinalen Erstreckungsrichtung der zweiten Schicht (14) beabstandet,
zumindest einen elektrischen Verbindungsbereich (17), und
wobei der zumindest eine elektrische Verbindungsbereich (17) in Kontakt mit der einen halbleitenden Schicht (13, 16) der ersten und zweiten Schicht (14) steht und eingerichtet ist, um mit einer elektrischen Energiequelle verbunden zu werden, und
wobei die erste halbleitende Schicht (13) und die zweite halbleitende Schicht (16) in einem Abstand voneinander angeordnet sind und eingerichtet sind, sich in Abhängigkeit von der auf das Sensorelement (10) ausgeübten Kraft selektiv zu kontaktieren, wodurch sich ein Widerstandswert des Sensorelements (10) ändert.

2. Das Sensorelement gemäß Anspruch 1, wobei die erste halbleitende Schicht (13) und die zweite halbleitende Schicht (16) zwei einander zugewandte Oberflächen ausbilden, welche in einem unbelasteten Zustand voneinander beabstandet sind und in Reaktion auf die auf das Sensorelement (10) ausgeübte Kraft in Kontakt gebracht werden können, und wobei ein quadratisches Maß, mit dem die beiden Oberflächen einander kontaktieren, ein Indikator für den Widerstandswert des Sensorelements (10) ist.

3. Das Sensorelement gemäß einem der vorhergehenden Ansprüche, wobei die Mehrzahl der elektrisch leitenden Abschnitte (19) auf der Trägerfolie (15) der zweiten Schicht (14) angeordnet ist und in Kontakt mit der halbleitenden Schicht (16) der zweiten Schicht (14) steht. Schicht (14), wobei sich die elektrisch leitenden Abschnitte transversal zur longitudinalen Richtung der zweiten Schicht (14) erstrecken.

4. Das Sensorelement gemäß einem der vorhergehenden Ansprüche, wobei eine Schicht, welche die Mehrzahl von elektrisch leitenden Abschnitten (19) aufweist, zumindest teilweise in der halbleitenden Schicht (16) der zweiten Schicht (14) eingebettet ist.

5. Das Sensorelement gemäß einem der vorhergehenden Ansprüche, wobei der elektrische Verbindungsbereich (17), welcher vorzugsweise als eine Schicht ausgebildet ist, ist zumindest teilweise in der halbleitenden Schicht (13) der ersten Schicht (11) eingebettet.

6. Das Sensorelement gemäß einem der vorhergehenden Ansprüche, wobei zumindest zwei erste und/oder zweite halbleitende Schichten (13, 16) in Bezug auf ihre longitudinale Erstreckungsrichtung parallel angeordnet sind, wobei die beiden ersten und/oder zweiten halbleitenden Schichten (13, 16) via den zumindest einen elektrischen Verbindungsbereich (17) miteinander verbunden sind.

7. Das Sensorelement gemäß einem der vorhergehenden Ansprüche, wobei die erste Schicht und die zweite Schicht (11, 14) zusammen mit der ersten halbleitenden Schicht und der zweiten halbleitenden Schicht laminiert sind, welche mittels einer Kohlenstoffzwischenschicht und/oder einen dazwischen angeordneten Klebstoff (18) voneinander getrennt sind.

8. Eine Sensoranordnung (100) zum Detektieren einer Kraft zwischen einem Fuß und einer stützenden Oberfläche, aufweisend:
eine Mehrzahl von Sensorelementen (10) gemäß einem der vorhergehenden Ansprüche, wobei die Sensorelemente (10) nebeneinander in einer zumindest im Wesentlichen gemeinsamen Ebene angeordnet sind,
eine Mehrzahl von elektrischen Leitungen (120), konfiguriert, um die individuellen Sensorelemente mit einem ersten und zweiten elektrischen Potential und/oder einer Auswertevorrichtung zu verbinden,
wobei die Sensoranordnung (100) zumindest eine periphere Kontur (101) aufweist, welche zumindest eine Kerbe (102) aufweist, die in Bezug auf die periphere Kontur (101) nach innen gerichtet ist und/oder die Sensoranordnung (100) zumindest eine periphere Kontur (101) mit zumindest einer Materialaussparung (103) aufweist, welche innerhalb der peripheren Kontur (101) angeordnet ist.

9. Ein Sensorsystem (200) zum Detektieren einer Kraft zwischen einem Fuß und einer stützenden Oberfläche, aufweisend:
zumindest ein Sensorelement (10) gemäß einem der Ansprüche 1 bis 7, das einen variablen Widerstand aufweist und eingerichtet ist, um ein elektrisches Detektionssignal in Reaktion auf die darauf ausgeübte Kraft bereitzustellen, und das mit einem ersten elektrischen Potential verbunden ist,
ein Widerstandsarray (210), eingerichtet zur elektrischen Beeinflussung des elektrischen Detektionssignals des Sensorelements, und mit einem zweiten elektrischen Potential verbunden,
einen Wandler (220), welcher zumindest einen Eingabekanal aufweist, welcher eingerichtet ist, um das elektrisch beeinflusste elektrische Signal des Sensorelements zu erhalten, und zumindest einen Ausgabekanal, um ein digitales Detektionssignal bereitzustellen, welches auf dem umgewandelten elektrischen Detektionssignal basiert, und
ein Datenverarbeitungsmittel (230), welches eingerichtet ist, um eine Schaltkreiskonfiguration eines oder mehrerer Widerstände des Widerstandsarrays zu bestimmen, welche zum Beeinflussen des elektrischen Detektionssignals verwendet werden.

10. Das Sensorsystem gemäß Anspruch 9, wobei das Datenverarbeitungsmittel (230) ferner eingerichtet ist, um die Schaltkreiskonfiguration des einen oder der mehreren Widerstände zu bestimmen, welche einen relativen Widerstands- oder Spannungswert betrifft.

11. Das Sensorsystem gemäß Anspruch 9 oder 10, wobei das Datenverarbeitungsmittel (230) ferner eingerichtet ist, durch Bestimmen der Schaltkreiskonfiguration des einen oder mehrere Widerstände, welche verwendet werden sollen, einen Messbereich des zu verwendenden Wandlers zu setzen, welcher Messbereich kleiner ist als eine vorbestimmte Auflösung des Wandlers (220).

12. Das Sensorsystem gemäß Anspruch 11, wobei das Datenverarbeitungsmittel (230) ist ferner eingerichtet, eine Charakteristik des detektierten Signals zu bestimmen und den Messbereich (MR) zwischen einem oberen Grenzwert (T2) und einem unteren Grenzwert (T1) zu setzen, wobei die Charakteristik (SC) des Detektionssignals zwischen dem oberen Grenzwert (T2) und dem unteren Grenzwert (T1) zumindest als quasi-linear bestimmt wird.

13. Das Sensorsystem gemäß Anspruch 12, wobei das Datenverarbeitungsmittel ferner eingerichtet, um die Obergrenze und/oder die Untergrenze des Messbereichs (MR) in Bezug auf eine gewünschte Empfindlichkeit des Sensorelements (10) zu setzen.

14. Ein Verfahren zum Detektieren einer Kraft zwischen einem Fuß und einer stützenden Oberfläche, wobei ein Sensorsystem (200) gemäß einem der Ansprüche 9 bis 13 verwendet wird, wobei das Verfahren aufweist:
Erhalten eines Kraft Detektionssignals von einem Sensorelement (10), Analysieren des Kraft Detektionssignals, um zumindest eine nichtlineare Charakteristik des Detektionssignales zu detektieren,
Erzeugen eines Konfigurationssignals, aufweisend eine Konfiguration von einem oder mehreren Widerständen eines Widerstandsarrays, welche verwendet werden sollen, um ein elektrisches Detektionssignal zu beeinflussen, auf welchem das Kraft Detektionssignals basiert, wobei die Konfiguration auf der Grundlage der detektierten nichtlinearen Charakteristik bestimmt wird, und
Bereitstellen des Konfigurationssignals, wobei das Konfigurationssignal dazu verwendet werden soll, die Konfiguration des Widerstandsarrays (210) zu konfigurieren.

15. Ein Computerimplementiertes Verfahren Zum Analysieren des Gangs eines Subjekts, aufweisend:
Bestimmen eines Zeitverlaufs einer Bodenreaktionskraft von zumindest einem Gangzyklus von einem Subjekt, wobei ein Sensorsystem (200) gemäß einem der Ansprüche 9 bis 13 verwendet wird,
Normalisieren des bestimmten Zeitverlaufs der Bodenreaktionskraft,
Vergleichen des normalisierten Zeitverlaufs mit einer Referenz,
Bestimmen des Ganges des Subjekts basierend auf einem Ergebnis des Vergleichs.

## Revendications

1. Élément de capteur (10) pour détecter une force entre un pied et une surface d'appui, comprenant :
une première couche (11) comprenant une feuille de support électriquement non conductrice (12) et une première couche semi-conductrice (13) s'étendant au moins partiellement le long d'une direction longitudinale de la première couche (11),
une seconde couche (14) comprenant une feuille de support électriquement non conductrice (15) et une seconde couche semi-conductrice (16) s'étendant au moins partiellement le long d'une direction longitudinale de la seconde couche (14),
une couche comprenant une pluralité de sections électriquement conductrices (19), dans laquelle la pluralité de sections électriquement conductrices (19) forment, lorsqu'elles sont considérées dans une direction d'épaisseur de l'élément de capteur, une forme de pavé de touches ou une forme de clavier avec les sections électriquement conductrices (19) en forme de bandes, et deux bandes adjacentes étant espacées l'une de l'autre dans la direction d'extension longitudinale de la seconde couche (14),
au moins une zone de connexion électrique (17), et
dans lequel la au moins une zone de connexion électrique (17) est en contact avec la couche semi-conductrice (13, 16) des première et seconde couches (14) et étant adaptée pour être connectée à une source d'énergie électrique, et dans lequel la première couche semi-conductrice (13) et la seconde couche semi-conductrice (16) sont agencées à distance l'une de l'autre et sont adaptées pour être sélectivement en contact l'une avec l'autre en fonction de la force appliquée à l'élément de capteur (10), en changeant ainsi une valeur de résistance de l'élément de capteur (10) .

2. Élément de capteur selon la revendication 1, dans lequel la première couche semi-conductrice (13) et la seconde couche semi-conductrice (16) forment deux surfaces dirigées l'une vers l'autre, qui sont espacées l'une de l'autre dans un état non chargé et, en réaction à la force appliquée à l'élément de capteur (10), peuvent être mises en contact, et dans lequel une mesure de superficie, avec laquelle les deux surfaces sont en contact l'une avec l'autre, est indicative de la valeur de résistance de l'élément de capteur (10).

3. Élément de capteur selon l'une quelconque des revendications précédentes, dans lequel la pluralité de sections électriquement conductrices (19) est agencée sur la feuille de support (15) de la seconde couche (14) et étant en contact avec la couche semi-conductrice (16) de la seconde couche (14), les sections électriquement conductrices s'étendant transversalement à la direction longitudinale de la seconde couche (14).

4. Élément de capteur selon l'une quelconque des revendications précédentes, dans lequel une couche comprenant la pluralité de sections électriquement conductrices (19) est au moins partiellement incorporée dans la couche semi-conductrice (16) de la seconde couche (14) .

5. Élément de capteur selon l'une quelconque des revendications précédentes, dans lequel la zone de connexion électrique (17), qui est de préférence formée comme une couche, est au moins partiellement incorporée dans la couche semi-conductrice (13) de la première couche (11) .

6. Élément de capteur selon l'une quelconque des revendications précédentes, dans lequel au moins deux premières et/ou secondes couches semi-conductrices (13, 16) sont agencées parallèlement par rapport à leur direction d'extension longitudinale, les deux premières et/ou secondes couches semi-conductrices (13, 16) étant connectées l'une à l'autre via la au moins une zone de connexion électrique (17).

7. Élément de capteur selon l'une quelconque des revendications précédentes, dans lequel la première couche et la seconde couche (11, 14) sont stratifiées ensemble avec la première couche semi-conductrice et la seconde couche semi-conductrice espacées l'une de l'autre par une couche de carbone intermédiaire et/ou un adhésif (18) agencé entre celles-ci.

8. Agencement de capteurs (100) pour détecter une force entre un pied et une surface d'appui, comprenant :
une pluralité d'éléments de capteur (10) selon l'une quelconque des revendications précédentes, les éléments de capteur (10) étant agencés adjacents les uns aux autres dans au moins un plan sensiblement commun,
une pluralité de lignes électriques (120), configurées pour connecter les éléments de capteur individuels à un premier et un second potentiel électrique et/ou un dispositif d'évaluation,
dans lequel l'agencement de capteurs (100) comprend au moins un contour périphérique (101) ayant au moins une encoche (102) qui est dirigée vers l'intérieur par rapport au contour périphérique (101) et/ou l'agencement de capteurs (100) comprend au moins un contour périphérique (101) ayant au moins un évidement de matière (103) qui est agencé à l'intérieur du contour périphérique (101).

9. Système de capteur (200) pour détecter une force entre un pied et une surface d'appui, comprenant :
au moins un élément de capteur (10) selon l'une quelconque des revendications 1 à 7, ayant une résistance variable et adapté pour fournir un signal de détection électrique en réaction à la force appliquée à celui-ci, et connecté à un premier potentiel électrique,
un réseau de résistances (210), adapté pour influencer électriquement le signal de détection électrique de l'élément de capteur, et connecté à un second potentiel électrique,
un convertisseur (220), ayant au moins un canal d'entrée, adapté pour obtenir le signal électrique influencé électriquement de l'élément de capteur, et au moins un canal de sortie pour fournir un signal de détection numérique basé sur le signal de détection électrique converti, et
des moyens de traitement de données (230), adaptés pour déterminer une configuration de circuit d'une ou plusieurs résistances du réseau de résistances utilisé pour influencer le signal de détection électrique.

10. Système de capteur selon la revendication 9, dans lequel les moyens de traitement de données (230) sont en outre adaptés pour déterminer la configuration de circuit de la ou des résistances associées à une valeur de résistance ou de tension relative.

11. Système de capteur selon la revendication 9 ou 10, dans lequel les moyens de traitement de données (230) sont en outre adaptés pour, en déterminant la configuration de circuit de la ou des résistances à utiliser, définir une étendue de mesure du convertisseur à utiliser, laquelle étendue de mesure est inférieure à une résolution prédéterminée du convertisseur (220).

12. Système de capteur selon la revendication 11, dans lequel les moyens de traitement de données (230) sont en outre adaptés pour déterminer une caractéristique du signal de détection et pour définir l'étendue de mesure (measuring range, MR) entre une limite supérieure (T2) et une limite inférieure (Tl), et dans lequel la caractéristique (SC) du signal de détection entre la limite supérieure (T2) et la limite inférieure (T1) est déterminée pour être au moins quasi linéaire.

13. Système de capteur selon la revendication 12, dans lequel les moyens de traitement de données sont en outre adaptés pour fixer la limite supérieure et/ou la limite inférieure de l'étendue de mesure (MR) associée à une sensibilité voulue de l'élément de capteur (10).

14. Procédé pour détecter une force entre un pied et une surface d'appui, en utilisant un système de capteur (200) selon l'une quelconque des revendications 9 à 13, le procédé comprenant de :
obtenir, à partir d'un élément de capteur (10), un signal de détection de force,
analyser le signal de détection de force pour détecter au moins une caractéristique non linéaire du signal de détection,
générer un signal de configuration, comprenant une configuration d'une ou plusieurs résistances d'un réseau de résistances qui doivent être utilisées pour influencer un signal de détection électrique sur lequel le signal de force de détection est basé, dans lequel la configuration est déterminée sur la base de la caractéristique non linéaire détectée, et
fournir le signal de configuration, dans lequel le signal de configuration doit être utilisé pour régler la configuration du réseau de résistances (210).

15. Procédé mis en oeuvre par ordinateur pour analyser l'allure d'un sujet, comprenant de :
déterminer une chronologie d'une force de réaction du sol d'au moins un cycle d'allure d'un sujet, en utilisant un système de capteur (200) selon l'une quelconque des revendications 9 à 13,
normaliser la chronologie déterminée de la force de réaction du sol,
comparer la chronologie normalisée à une référence, et
déterminer l'allure du sujet sur la base d'un résultat de ladite comparaison.
